# EUROPEAN PATENT SPECIFICATION

(11) **EP 3 522 947 B1**
(45) Date of publication and mention of the grant of the patent: **19.02.2025**
(21) Application number: 17787358.5
(22) Date of filing: 06.10.2017
(51) Int. Cl.: A61M 60/178, A61M 60/216, A61M 60/865

(54) **TRANSCATHETER DEVICE FOR THE DELIVERY OF INTRACORPOREAL DEVICES**
TRANSKATHETERVORRICHTUNG ZUR ABGABE VON INTRAKORPORALEN VORRICHTUNGEN
DISPOSITIF TRANSCATHÉTER POUR LA POSE DE DISPOSITIFS INTRACORPORELS

(30) Priority: 07.10.2016 US 201615288642; 07.10.2016 US 201615288738
(43) Date of publication of application: 14.08.2019
(73) Proprietor: Nuheart AS, 5528 Haugesund (NO)
(72) Inventor: TUSETH, Vegard, 5019 Bergen (NO); PATTERSON, Shawn, Minneapolis Minnesota 55419 (US); HAARSTAD, Philip, Chanhassen Minnesota 55317 (US)
(74) Representative: Jacob, Reuben Ellis
(86) International application number: PCT/EP2017/075561
(87) International publication number: WO 2018/065610

(56) References cited:
- EP-A1- 3 410 986
- US-A1- 2006 155 158
- US-A1- 2012 172 654
- US-A1- 2015 005 570
- US-A1- 2015 119 633
- US-A1- 2015 223 839
- US-A1- 2016 175 501

## Description

### FIELD OF THE INVENTION

The present invention generally relates to the field of medical devices and surgery devices. More specifically, the invention relates to a transcatheter system and corresponding devices. The present invention is particularly useful for the delivery and implantation into the body of a patient of medical devices, such as mechanical circulatory support devices, but also has a wider variety of applications.

The present invention is particularly useful in the context of minimally invasive transcatheter and/or percutaneous procedures, such as those described by the Applicant in US patent applications US 14/335125, US 14/335142, US 14/991662, US 14/991675, US 14/984998 and US 14/985030, and any U.S. published applications for the same.

### BACKGROUND

Examples of mechanical circulatory support systems (MCS) include ventricular assist devices (VADs). A VAD is a mechanical pumping device capable of supporting heart function and blood flow. Specifically, a VAD helps one or both ventricles of the heart to pump blood through the circulatory system. Left ventricular assist devices (LVAD), right ventricular assist devices (RVAD) and biventricular assist devices (BiVAD) are currently available. Also, circulatory support systems may include cardiopulmonary support (CPS, ECMO), which provide means for blood oxygenation as well as blood pumping. Such devices may be required during, before and/ or after heart surgery or to treat severe heart conditions such as heart failure, cardiopulmonary arrest (CPA), ventricular arrhythmia or cardiogenic shock. Examples of such devices can be found in US patent applications US2016/175501A1 and US2006/155158A1.

Traditionally, VADs are fitted during open-heart surgery through an incision in the chest and the procedure involves puncturing the apex of the left ventricle to re-route blood from the ventricle to the aorta through an external pump. An example of a device used in a surgical VAD is HeartMate II^{™}. Such surgical procedures are clearly invasive and unsuitable for weaker and vulnerable patients as they involve a greater recovery time and carry the risks of infection and trauma. This is particularly the case in the treatment of children for whom existing surgical equipment and devices are comparatively bulkier and more invasive, and a reduction of the size of the equipment is often difficult if not impossible in view of the equipment and procedure involved. Furthermore, these devices require the intervention from a team of skilled surgical staff in a hospital environment and are therefore less available and costly.

More recent procedures are non-surgical and involve the insertion of a VAD through a small incision made at the groin of the patient. A popular version of such so-called percutaneous VAD is the TandemHeart^{™} device. A tube is introduced through an incision adjacent the groin of the patient and advanced along the femoral vein and inferior vena cava, across the intra-atrial septum and into the left atrium so that oxygenated blood from the left atrium is fed into a pumping device located outside the patient's body and recirculated through an outflow tube into the femoral artery. Although this device has shown promising results, it only provides short-term support (up to two weeks) and is unsuitable for long-term treatments. The external pump is bulky and requires the patient's immobilization for as long as the device is fitted. Furthermore, there is a risk of life-threatening infection around the groin incision, which remains open during the treatment, and of considerable bleeding from a major artery. In addition, the tube of the TandemHeart^{™} ends in the left atrium from which blood is pumped out and led outside the patient's body. This type of blood inlet system can potentially become hindered, if not blocked, if surrounding tissues are accidentally sucked in, thereby resulting in a loss of efficiency.

Another popular percutaneous VAD is the Impella^{™} device, which is inserted into the femoral artery and descending aorta. The Impella^{™} device comprises an elongated end, which is implanted across the natural aortic valve, with a blood inlet placed in the left ventricle and a blood outlet above the aortic valve. A pump circulates blood from the inlet to the outlet. The driveline is externalised through the femoral artery during use and the same limitations apply as with TandemHeart^{™} and other current percutaneous MCS systems. This device is approved to provide support for up to a week. There is therefore a need for a device with reduced risk of infection and bleeding and increased mechanical stability which can be used as part of a short-term "bridge to recovery" treatment or as a long-term treatment including patient mobilisation. In addition, the efficiency of the pump is limited because it is not possible to insert a pump of the size required to provide a suitable blood flow using percutaneous arterial access. Presently, the problem of limited pump capacity and duration with percutaneous MCS is solved either by inserting larger intracorporeal pumps surgically or by choosing an extracorporeal pump, with all the potential problems as described above.

Known mechanical circulatory support systems are positioned within the patient directly though open-heart surgery or indirectly for example using transcatheter methods in which the device is pushed to its intended position. Such a transcatheter method is described by the Applicant in PCT/EP2015/055578. These methods involve the implantation of a guide wire extending for example from the patient's groin area, along the femoral vein, up the inferior vena cava into the right atrium, through the atrial wall into the left atrium, through the atrial roof and aortic wall into the aorta, so that the proximal end of the guide wire is outside the groin area of patient and the distal end is in the aorta. The guide wire assists in the subsequent positioning of catheters and/or sheaths through which the various devices are pushed to their target positions.

It has however been observed that the ability to accurately guide and position the devices is affected by factors such as the target position, for example, where the device is to be moved through or positioned across anatomical walls, and/or at awkward angles. Safety will of course also be a concern. The implantation process of the devices requires the insertion and manipulation of delivery systems and the puncture of anatomical walls; the VAD device must be delivered and implanted and functions in a safe, secure and atraumatic manner. There may also be some issues with the coupling between the guide wire and the device to be delivered. Ideally, the guide wire is positioned slidably and centrally within the device. Depending on the structure of the device, this central positioning is not always achievable and the device is pushed alongside the guide wire in the catheter. This central positioning of the guide wire may also affect and possibly compromise the design and/or function of the device, for example the hydraulic design and function of a pump device. This potentially can result in a less accurate, and therefore less safe, device delivery process.

### SUMMARY OF THE INVENTION

It is an object of the invention to at least alleviate the above-mentioned disadvantages, or to provide an alternative to existing products.

According to a first aspect of the invention, there is provided an intracorporeal device for delivery into a patient, wherein said intracorporeal device having a proximal end and a distal end and comprising means for detachably coupling its distal end with a transcatheter delivery device, and further comprises an integrated expandable distal component, said distal component being able to change configuration from a delivery configuration to a working configuration.

The present invention provides an intracorporeal device arranged and configured to be connected to a delivery device, in particular a delivery device capable of pulling the intracorporeal device into position, and to be detached after delivery, use and/or implantation.

The delivery configuration is a configuration suitable for transcatheter delivery. The delivery configuration is configuration in which the intracorporeal device can be coupled to the delivery device; whereas in the working configuration, the intracorporeal device is detached from the delivery device.

In one example, the delivery configuration may be a folded configuration and the working configuration may be a deployed configuration. However, the opposite configurations are also envisaged, namely a deployed delivery configuration and a folded delivery configuration. Similarly, in a further example, the delivery and working configurations may be retracted and extended configurations. The distal component may also be arranged and configured to change from a working position into a retrieval configuration.

The distal component may be able to change configuration from a working configuration to a retrieval configuration. In this case, the retrieval configuration may be a configuration suitable for transcatheter retrieval. The retrieval configuration may be a configuration in which the intracorporeal device can for example be coupled to the retrieval device; whereas in the working configuration, the intracorporeal device is detached from the retrieval/delivery device.

The present invention is described herein with reference to a working configuration in which the distal component is deployed and a delivery or retrieval configuration in which the distal component is folded or extends substantially longitudinally from the distal end of the intracorporeal device. The opposite is also envisaged within the context of the invention, i.e. a delivery or retrieval configuration in which the distal component is deployed and/or a working configuration in which the distal component is folded or extends substantially longitudinally from the distal end of the intracorporeal device.

The integrated distal component may be integrally formed with the intracorporeal device for ease of manufacture. In addition, the intracorporeal device with its integral distal component may be delivered and implanted safely as a single device. Alternatively, the distal component may be connected to the intracorporeal device. This arrangement allows for more versatility and flexibility in the delivery and implantation procedures. In such an example, said connection is effected prior to insertion into the patient, and which may be during the manufacturing process. Although the distal component may be connected to the intracorporeal device intracorporeally, it is preferable to minimise the number of manipulations within the patient.

In an example, the distal component may be movably connected to the intracorporeal device so that it can be positioned in a delivery configuration and in a working configuration. For example, the distal component can be hingedly connected to the intracorporeal device or the distal component comprises or consists of a shape-memory material, which enables the transition between a delivery configuration and a working configuration.

Preferably, in the working configuration, the integrated expanded distal component is arranged and configured to secure the intracorporeal device to one or more anatomical walls. In this embodiment, the distal component serves the dual purpose of detachably coupling the intracorporeal device to a delivery device and to secure the intracorporeal device to its target site.

Preferably, in the working configuration, the integrated expanded distal component is or acts as a flow diffusor. In this embodiment, the distal component serves the dual purpose of detachably coupling the intracorporeal device to a delivery device and to improve fluid flow and dynamics. This is particularly relevant when the intracorporeal device comprises a pump. The diffuser is arranged and configured to disperse and spread the fluid exiting from the pump in order enhance fluid flow and improve control of the fluid flow. More generally, a distal component (which is, comprises or acts as) a distal diffuser may assist in optimizing fluid dynamics in order to optimize blood flow out of the pump for optimum efficiency and minimal thrombus or blood damage.

Within the context of this invention, it is also envisaged that the diffuser be alternatively located within the main body of the pump or at its proximal. However, optimum results are achieved when the diffuser extends from or is coupled to the distal or distal most end of the pump so that the flow of fluid is enhanced as it exits the pump. It is preferred to avoid positioning elements, such as a diffuser, within the main body of the pump, as they could potentially hinder the fluid flow and/or narrow the fluid path within the pump.

Preferably, the integrated expandable distal component comprises a plurality of expandable arms, blades and/or mesh. When the integrated expandable component is a diffusor, the general contour of the deployed arms/blades allows the flow to be enhanced. When the integrated expandable component is a connector, the arms and/or blade can deploy outwardly to lie against the anatomical wall of the second compartment (i.e. the receiving compartment).

The distal component in its working configuration may partially curved away from the anatomical wall in order to minimise tissue growth or trauma. Alternatively, the distal component in its working configuration may extend away from the anatomical wall and into the second compartment. For example the distal component may extend substantially as a truncated inverted cone or funnel from the distal end of the intracorporeal device.

Preferably, the integrated expandable distal component comprises a membrane extending between the arms and/or blades. The distal component may comprise means for partially or completely sealing the area between the arms or blades, for example covering, coating or other sealing means. When the integrated expandable component is a diffusor, then the membrane improves the diffusor efficiency. The membrane increases the surface area of the diffusor contacting the fluid and therefore increases the efficiency of the diffusor. When the integrated expandable component is a connector, the membrane may act as an additional anchor but more importantly as an additional structural support and protection for the anatomical wall(s). In addition, in its working configuration, the distal component may be not fully inserted across the anatomical walls, and the sealing means will seal the gap between the distal end of the intracorporeal device and the anatomical walls (or the gap between the intracorporeal device and the primary connector means) in order to achieve minimal blood leakage The membrane preferably comprises or consists of a biocompatible flexible material, which extends between the arms and/or blades (e.g. like an umbrella).

Within the context of this invention, the integral distal component may consist of a membrane (i.e. without any arms and/or blades). This provides for a flexible diffuser/connector which is unlikely to damage the surrounding tissues upon deployment. In this embodiment, the membrane may be made of a shape memory material, or of a flexible material which will adopt the working configuration owing to the fluid flowing out of the pump.

Preferably, the intracorporeal device further comprises means for pushing the integrated expandable distal component into its working configuration. It is envisaged that the integrated expandable distal component may be pushed, pulled and/or otherwise assisted into its working configuration. It is also envisaged means for pushing, pulling and/or otherwise assisting the integrated expandable distal component back into its retrieval/delivery configuration.

In an embodiment, such means comprises an inflatable balloon positioned at the distal end of the intracorporeal device. The intracorporeal device may comprise a balloon, which is in a deflated state in the delivery configuration. Upon inflation, the balloon forces the deployment of (the arms and/or blades of) the integral expandable distal component. The balloon may be positioned centrally with the arms and/or blades of the integrated expandable distal component positioned circumferentially around the balloon.

The inflatable balloon may be provided as part of the intracorporeal device or as part of a delivery device. An inflation line in fluid communication with the inflatable balloon may be provided as part of the intracorporeal device or as part of a delivery device. The inflatable balloon may be detached from the intracorporeal device and/or the delivery device after use.

A delivery device may be provided which comprises an elongate flexible member and an inflatable balloon at one end thereof, i.e. the end of the delivery device which is intended to be coupled to the intracorporeal device. The end of the delivery device comprising the inflatable balloon is coupled to the end of the intracorporeal device comprising the integral expandable component. Once the intracorporeal device is suitably positioned at the target site, the balloon is inflated so as to assist the deployment of the integral expandable component. When the integral component is suitably expanded, the delivery device (and its deflated balloon) may be detached from the intracorporeal device and removed from the patient.

In an embodiment, the coupling means may comprise an integral coupling component comprising a cap or a sheath. In this instance, the cap or sheath is integrally formed with the intracorporeal device, preferably adjacent or at the distal end of the device. In one example, the cap or sheath is arranged and configured such that it partially or wholly surrounds a distal expandable component. One of its functions is to keep the expandable component in a non-expanded configuration. For example, if the expandable component comprises plurality of expandable arms/blades, these would be kept together in a delivery configuration under the cap or sheath. In order to release the expandable arms/blades, the cap/sheath may be removed in any suitable manner, for example torn or pulled back.

Preferably, the intracorporeal device comprises a detachable coupling component, said distal component being able to be expanded from a delivery configuration to a working configuration. In this embodiment, "detachable component" means that the component is attached to the intracorporeal device before and during the delivery process and can be detached after delivery/implantation. "Coupling component" means that the component is destined to couple the intracorporeal device to the delivery device. Thus, in this embodiment, the coupling means is not an integrated distal end, but can be detached from the intracorporeal device.

Preferably, the detachable component comprises a cap and/or a sheath, similar to the integral cap or sheath, but which in this embodiment is detachable. Upon delivery of the intracorporeal device, the cap/ sheath can be detached from the intracorporeal device, thereby releasing the arms/blades into a working configuration, and be removed from the patient.

In one example, the detachable component may be attached to the intracorporeal device by any suitable means, including screw means, tabs, hooks, tearline and the like.

Preferably, the detachable coupling component is secured to the delivery device. The cap or sheath can be detachably coupled to the intracorporeal device but secured to the delivery device. Prior to or during the delivery process, the intracorporeal device is coupled to a delivery device, more specifically the distal component of the intracorporeal device is coupled to a cap or sheath. The cap or sheath is secured to or integrally formed with the delivery device. Once the intracorporeal device is suitably delivered and/or implanted, the cap or sheath is detached from the distal component of the intracorporeal device and removed by pulling the delivery device out of the patient.

In one example, the distal component is made or comprises a resilient material such that, in a first configuration, the distal component is deployed and in a second configuration, the distal component is folded. In another example, the distal end of the distal component is contained within the detachable coupling component (e.g. cap or sheath) and attachment is achieved by the mechanical force of the resilient distal component folded within the detachable coupling component.

In another example, the detachable coupling component is secured to the delivery device. During the delivery process, the coupling component secures the intracorporeal device to the delivery device. Once the intracorporeal device is suitably positioned, the detachment of the coupling component enables the integral expandable component to deploy into its working configuration. For example, the integral expandable component may comprise a plurality of arms which are hingedly connected to the distal end of the intracorporeal device. The arms may be substantially L-shaped so that one end of the arms can be deployed outwardly, whilst the other end is arranged and configured to be interlocked into a coupling position with the delivery device or to be used as a lever by pulling the delivery device to deploy the arms into a working position.

Within the context of this invention, the term "intracorporeal" means inside the body of a patient. For example, an intracorporeal device is a device which is destined for use and/or implantation inside the body of a patient. The term "extracorporeal" means outside the body of a patient. For example, an extracorporeal connection is a connection which takes place outside the body of the patient. It is also envisaged that a device comprises an intracorporeal part and an extracorporeal part.

The term "transcatheter" includes percutaneous, trans-atrial, trans-femoral (through the leg), trans-apical (in the chest between the ribs), trans-aortic (in the upper chest), trans-iliac, trans-atrial and trans-axillar. Preferred embodiments are percutaneous systems, devices and methods.

The term "percutaneous" is used with reference to any medical procedure where access to inner organs or other tissue is done through a puncture and/or incision through the skin (and/or the vascular system) for example into the circulatory system, as opposed to an open surgery procedure. Thus, a percutaneous method involves the percutaneous delivery of elements and may involve an incision (for example with a scalpel) to enable percutaneous delivery. In a preferred embodiment, the method provides transcardiovascular delivery of one or more devices for establishing fluid communication between anatomically separate but adjacent thoracic organs, after gaining access to the vascular system by a puncture or incision. The puncture or incision may be made at various sites where intravascular access is possible, for example in the groin, axilla, chest or abdomen.

Within the context of the invention, any of the devices and systems described herein can be used with any of the methods described herein, said methods not forming part of the claimed invention.

The devices, systems and methods have been described above in connection to the delivery of an intracorporeal device to an intracorporeal target site. However, it is envisaged within the context of the invention that the devices, systems and methods described are used for the purpose of retrieving an intracorporeal device from its implantation site.

### BRIEF DESCRIPTION OF THE DRAWINGS

The invention will be further described with reference to the drawings and figures, in which
Figure 1 illustrates a transcatheter delivery method and system;
Figure 2 illustrates an anterior device delivery pathway;
Figure 3 illustrates a posterior device delivery pathway;
Figures 4a to 4c are schematic representations of an intracorporeal device;
Figure 5 is a schematic representation of an intracorporeal device and delivery devices;
Figure 6 is a schematic representation of an intracorporeal device and delivery devices;
Figures 7a and 7b are schematic representations of a first deployment mechanism for use as herein described;
Figures 8a and 8b are schematic representations of a second deployment mechanism for use as herein described;
Figure 9 is a schematic representation of an intracorporeal device and delivery device;
Figures 10a to 10d are schematic representations of delivery devices;
Figures 11a to 11c are schematic representations of delivery devices;
Figures 12a and 12b are schematic representations of a catheter or sheath with a radial separation line;
Figure 13 and figures 14a to 14 c are schematic representations of a deployment process in a method as herein described;
Figures 15a to 15d illustrate the delivery and implantation process of an intracorporeal device using a delivery sheath with a radial separation mechanism;
Figures 16a to 16e are schematic representations of an intracorporeal device according to the present invention and a delivery device as herein described;
Figures 17a to 17c are schematic representation of a guide wire for use in the process herein described or together with the devices according to the present invention;
Figure 17d illustrate a catheter comprising a curved tip for use as herein described; ; and

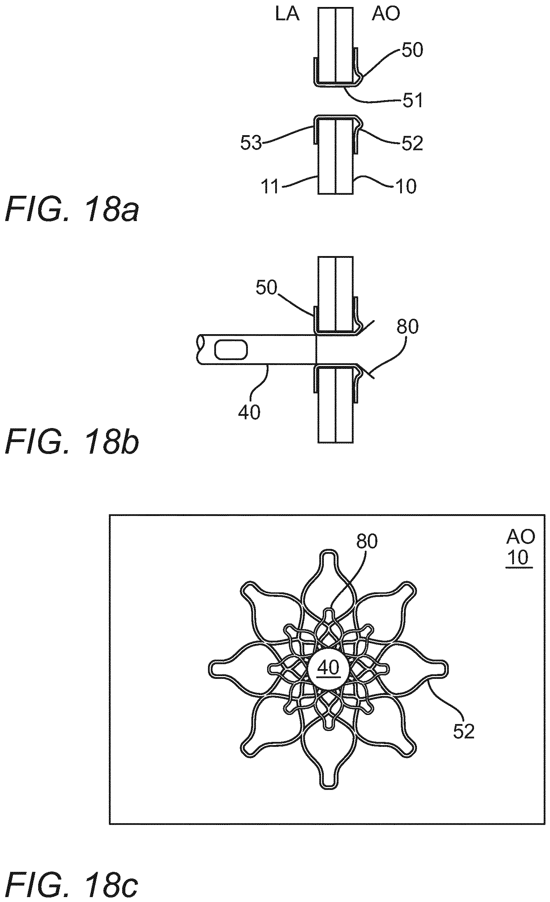

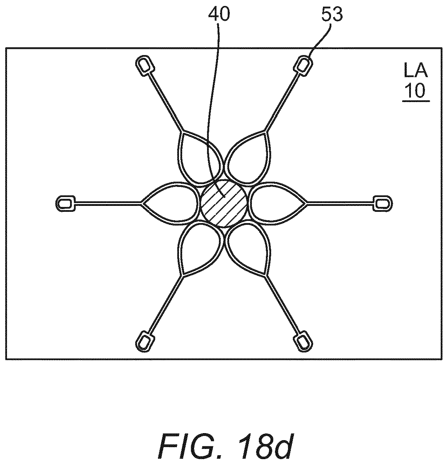

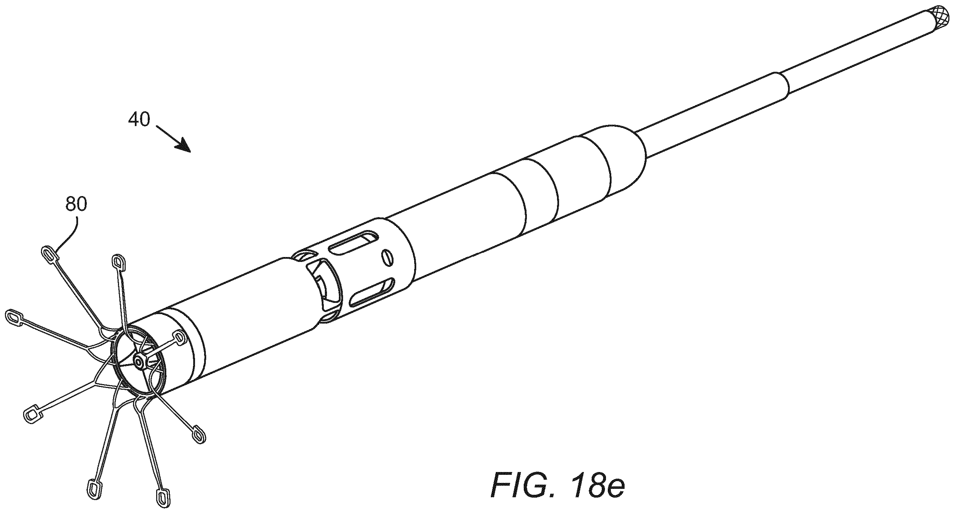

Figure 18a illustrates a primary connector means positioned in a working configuration across two anatomical walls; figure 18b illustrates the secondary connector means coupled in a working configuration with the primary connector means; figure 18c is a schematic view of the primary and secondary connector means from the distal anatomical compartment (e.g. the aorta), figure 18d is a schematic view of the primary connector means and the intracorporeal device from the proximal anatomical compartment (e.g. the left atrium); figure 18e is a schematic view of an intracorporeal device comprising a distal component or secondary connector means.

### DETAILED DESCRIPTION

The invention is described by way of examples, which are provided for illustrative purposes only. These examples should not be constructed as intending to limit the scope of protection that is defined in the claims. For example, although various aspects have been described with respect to the heart and the circulatory system, this is not intended to be limiting, and is merely performed to provide an example of implementation. Aspects disclosed herein may be utilised in any medical device implantable within the human body, for example in the cardiovascular system, respiratory system, gastric system, neurological system, and the like, some examples including implantable pumps and drug delivery pumps. As used herein, the term "means" can be equivalently expressed as, or substituted with, any of the following terms: device, apparatus, structure, part, sub-part, assembly, sub-assembly, machine, mechanism, article, medium, material, applicant, equipment, system, body or similar wording.

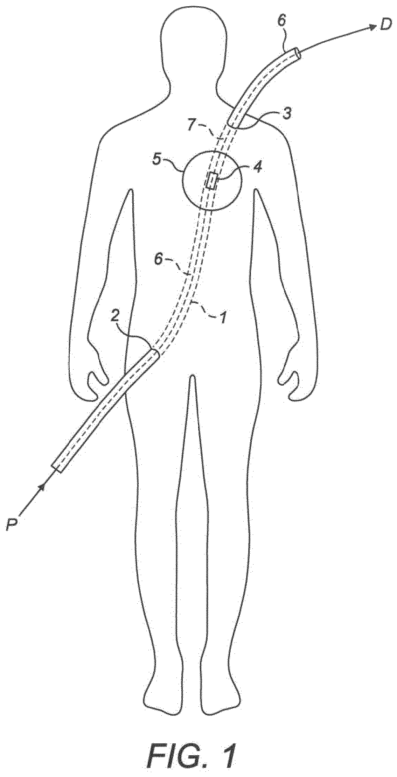

Referring to figure 1, there is illustrated a transcatheter delivery system and method including a intracorporeal device delivery pathway 1 extending from an entry point 2 into a patient to an exit point 3 from the patient. In this transcatheter method, an intracorporeal device 4 is delivered to a target site 5 through a delivery catheter or sheath 6. The intracorporeal device 1 is detachably connected to a device delivery system comprising a first delivery device 6 extendable at least between the entry point 2 to the proximal end of the intracorporeal device 4 and a second delivery device 7 extendable at least between the distal end of the intracorporeal device 4 and the exit point 3. The proximal end P and the distal end D are defined relative to the direction of insertion of the delivery device, i.e. in the direction of the entry point towards the exit point.

In the embodiment shown in figure 1, access into the patient's body is created through a puncture or incision in the groin area and the entry point 2 is into the femoral vein or artery. Exit from the patient's body is created through a puncture or incision in the neck area with an exit point 3 from the jugular vein or in the clavicle area with an exit point 3 from a subclavian artery.

The present invention will be described in the context of the implantation of an intracorporeal device 4 for establishing fluid communication between two anatomical compartments for example the left atrium LA and the aorta AO, wherein the target implantation site is across two separate anatomical walls 10, 11, in this example the roof of the left atrium and the aortic wall. However, it is understood that the present invention can be used for different procedures at different target sites.

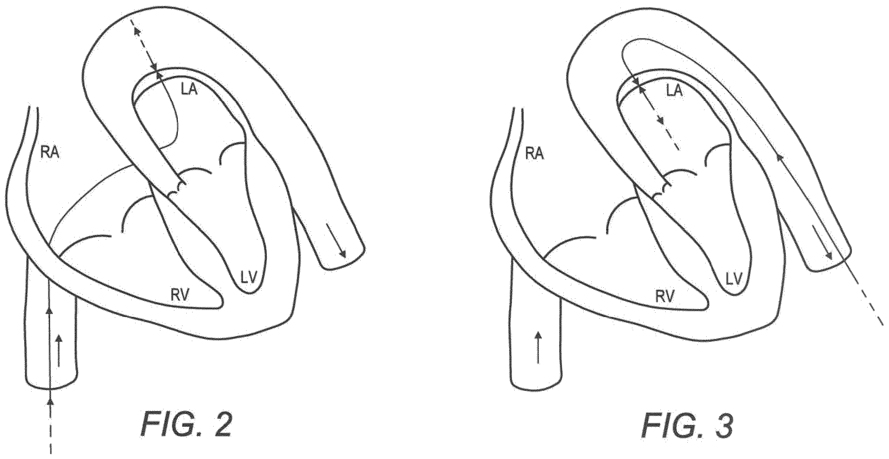

The device delivery pathway 1 extends at least between the entry point 2 and the exit point 3, but can also extend beyond the entry point 2 and the exit point 3. It is known to insert a device for intracorporeal use and/or implantation via the femoral, subclavian or jugular access. In these techniques, the device is inserted and pushed through the circulatory system up to the delivery site, for example the heart for a ventricular assist device. In the Applicant's own PCT/EP2015/055578, the devices are pushed through a puncture in the atrial septum, then delivered across the roof of the left atrium and the aortic wall in a so-called anterior delivery method (Figure 2). It is also possible to insert a device via a posterior pathway (Figure 3). For example, the devices can be inserted through the descending aorta into the left atrium and then delivered across the roof of the left atrium and the aortic wall.

In US 14/991662 and US 14/991675, the Applicant describes a connector comprising a neck, a first set of arms extending from a first end of the neck and a second set of arms extending from the second end of the neck. In an anterior delivery method, the delivery sheath passes through the atrial septum to the left atrium and into the aorta. In a posterior delivery method, the delivery sheath passes through the wall of the ascending aorta and the roof of the left atrium, so that the first arms deploy and lay against the roof of the left atrium as they exit the catheter, followed by the second arms against the wall of the ascending aorta. The posterior pathway is advantageous in that it presents fewer obstacles (e.g. anatomical walls to be punctured, proximity to the coronary artery and mitral and aortic valve in the anterior pathway). Depending on the structure and dimensions of the intracorporeal device 4, it can sometimes be difficult in the anterior method to accurately achieve the required positions and angles; whereas the posterior pathway provides more room to manoeuver so that larger devices can be implanted. Finally, the anterior pathway is more commonly used for physiological reasons but there is a risk of potential blood clots and strokes. This risk can be minimised by inserting posteriorly. The present invention can make use of delivery pathways such as anterior and posterior pathways.

The intracorporeal device 4 according to the present invention has a proximal end 4P and a distal end 4D, also defined relative to the direction of insertion, and means for detachably coupling its distal end 4D with a transcatheter delivery device so that the intracorporeal device 4 can be pulled in the direction of insertion. As discussed above, known methods involve pushing the intracorporeal device through a delivery sheath to the target site 5. By contrast, the method herein described alternatively or additionally involves pulling the intracorporeal device 4 to the target site; and the intracorporeal device 4 according to the present invention comprises means for being pulled to the target site 5. Most preferably, the method herein described enables the practitioner to both push and pull the intracorporeal device 4 (so that the intracorporeal device can be manipulated from both proximal and distal ends or from both the entry and the exit points into the patient); and the intracorporeal device 4 according to the present invention comprises means for being both pushed and pulled. This may be achieved by a number of different interconnections between the intracorporeal device 4 and the transcatheter delivery device. Within the context of the present invention, the intracorporeal device 4 may be inserted through and/or manipulated from a number of different access and exit points. The intracorporeal device 4 may be inserted forward or backward, relative to its target in-use position. Therefore, the various interconnections described herein may be used either at the distal end of the intracorporeal device 4, or both at the distal end and the proximal end of the intracorporeal device 4 (i.e. distal end 4D of the intracorporeal device 4 to proximal end of second delivery device 7; and proximal end 4P of the intracorporeal device 4 to distal end of the first delivery device 6). When the intracorporeal device 4 comprises an interconnection at its distal end and another interconnection at its proximal end, both interconnections can be the same or different. Similarly, the delivery devices described herein may be used as a first proximal delivery device 6 and/or as a second distal delivery device 7.

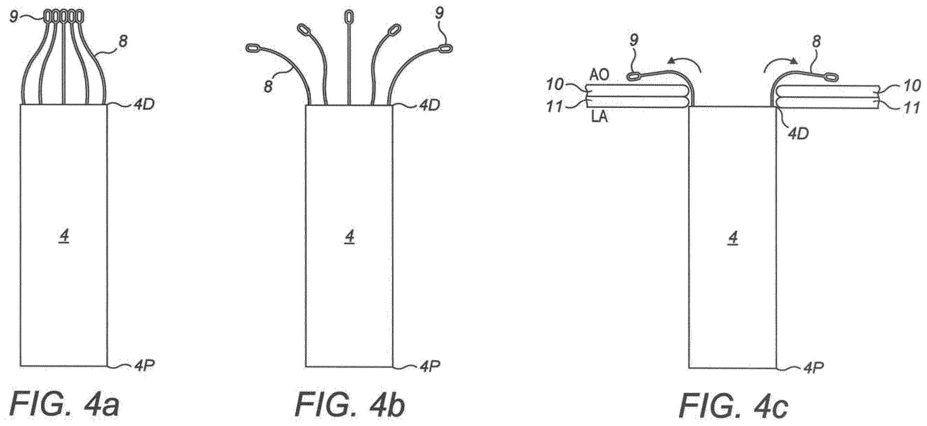

In an embodiment illustrated in figures 4a to 4c the intracorporeal device 4 comprises a housing with a proximal end 4P and a distal end 4D. The housing comprises a pump to regulate fluid flow between the left atrium LA and the aorta AO. The intracorporeal device 4 comprises a plurality of arms or blades 8 which are preferably integrally formed or secured to its distal end 4D. In a delivery (or retrieval) configuration as illustrated in figure 4a, the distal ends of the arms 8 are gathered together so that the arms 8 are folded and the device 4 fits in a delivery catheter. In a working configuration as illustrated in figures 4b and 4c, the arms 8 are released and deployed so that the unfolded arms 8 can rest partially or wholly against an anatomical wall. In this embodiment, the arms 8 serve the dual purpose of connecting the intracorporeal device 4 to the delivery device and acting as a connector or anchor. This intracorporeal device 4 is particularly suited to a target site across one or more anatomical walls 10, 11, as the integral arms 8 can secure the intracorporeal device 4 across said anatomical walls 10, 11, and support and protect said anatomical walls 10, 11. The plurality of arms 8 may comprise a set of short arms and a set of long arms (as described in the Applicant's US 14/991662 and US 14/991675) to ensure a smooth and atraumatic release and implantation of the intracorporeal device 4.

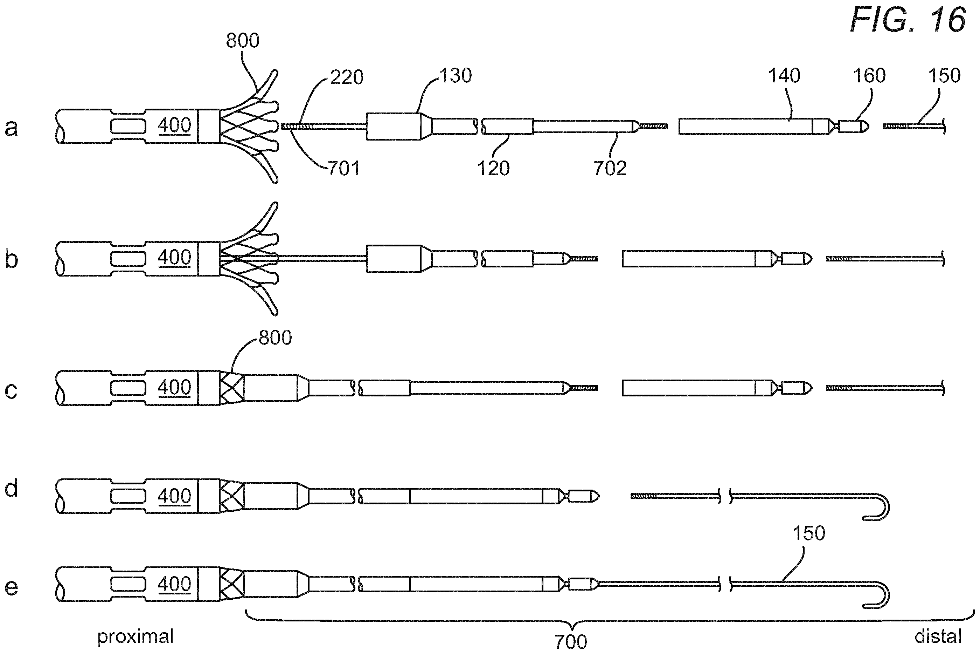

In a preferred embodiment, the intracorporeal device 4 comprises a plurality of arms or mesh 80, 800 extending from its distal end. The arms or mesh 80,800 are made of a resilient material such that, in a working configuration (see for example figures 16a and 16b), the arms 80,800 are deployed and in a delivery configuration (see for example figures 16 c, 16d, 16e), the arms 80,800 are folded and contained within the receiving portion 13, 130 by mechanical force owing to the resilience of the arms 80,800.

Advantages of this arrangement include firstly, that the flexible arms allow for the intracorporeal device to be centrally positioned; secondly, the arms allows for flexible and more versatile docking suitable for different anatomies; thirdly, the arms are self-expanding so that they will naturally expand into place without the need to exert pressure; fourthly, the flexible arms can be passively collapsed or folded during the retrieval procedure.

With respect to the third advantage, it is crucial that the procedure is as atraumatic as possible, and that little pressure is exerted onto the patient's anatomical walls in order to avoid injury, tear and/or blood loss. The self-expanding arms are collapsed when contained in a delivery catheter which is advanced adjacent the target implantation site. Once optimum positioning is achieved, the arms are pushed out of the delivery catheter and will naturally expand into their working configuration. There is therefore no need to apply pressure to push the arms across the anatomical walls or through a primary connector means 50 and the trauma risk is minimised. With respect to the fourth advantage, retrieval of the arms 80,800 from the anatomical walls, or disconnection from the primary connector means 50 is achieved in a simple and natural manner. As the arms of the intracorporeal device are pulled back and out of the primary connector means 50, the arms will naturally fold inwardly as they pass through the neck of the primary connector means 50. They will fold enough so as to fit for example in a retrieval device or retrieval sheath or catheter.

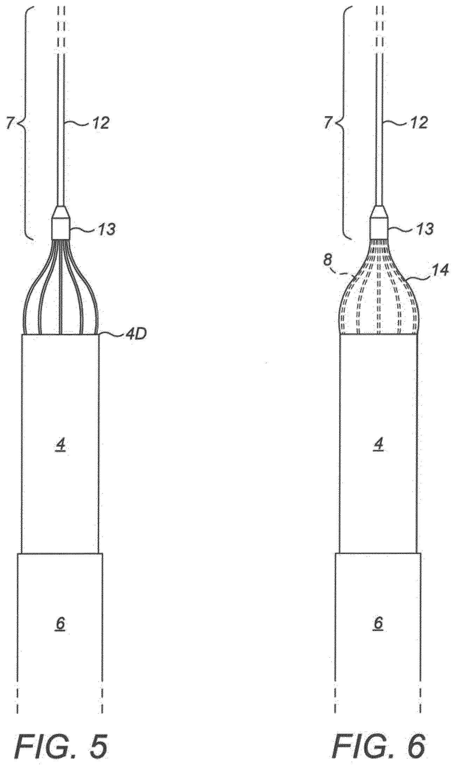

In figure 5, the distal delivery device 7 intended to pull the intracorporeal device 4 comprises an elongate flexible member, in this case a wire or cable 12 and a proximal portion for receiving the distal end of the arms 8. The wire or cable 12 is rigid enough to enable accurate guiding of the intracorporeal device 4, but flexible enough to avoid injury to the patient. The receiving portion 13 is cup- or bowl-shaped. The receiving portion 13 is preferably detachably connected to the arms 8, so as to be attached during delivery and detached after successful delivery. The receiving portion 13 may comprise internal connection means for detachably connecting to the arms 4. Upon detachment of the receiving portion 13 from the arms 8, the arms 8 are released and can change from their delivery configuration to their working configuration. The arms 8 may be retained within the receiving portion 13 through tension. The arms 8 may comprise eyelets 9 to facilitate connection to the receiving portion 13. The eyelets also increase the surface area of the arms 8 and provide additional support to the anatomical walls 10, 11.

In figure 6, the distal delivery device 7 comprises an elongate flexible member, in this case a wire or cable 12, a receiving portion 13 and a proximal sheath or cap 14. The sheath or cap 14 is arranged and configured to cover the folded arms 8 during the delivery process. The sheath or cap 14 is made of a bio-compatible material such as a membrane, fabric of the like. The sheath or cap 14 is secured either to the distal delivery device 7 or to the intracorporeal device 4. The sheath or cap 14 may be detachably connected to the distal delivery device 7 or to the intracorporeal device 4. For example, the sheath or cap 14 may be made of an elastic or resilient material so as to be connected to the distal end of the intracorporeal device 4; the sheath or cap 14 may be made of a tearable material or may comprise a suitably positioned tear line; the sheath or cap 14 and the distal delivery device 7 or to the intracorporeal device 4 may comprises complementary detachable connections means, such as tabs, screwing means, hooks, snares and the like. The distal delivery device 7 may or may not comprise a receiving portion 13. The distal ends of the arms 8 may be detachably connected to the elongate flexible member 12 by any other suitable means.

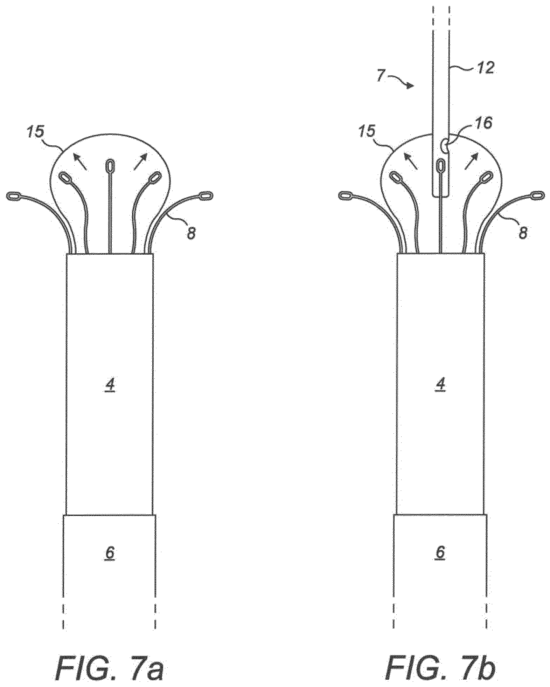

In figure 7a, the intracorporeal device 4 comprises an inflatable balloon 15 intended to assist the deployment of arms 8. The balloon 15 is arranged and configured so that, upon inflation, the balloon 15 pushes the arms 8 outwardly into their working configuration. The intracorporeal device preferably comprises an inflation line (not shown) for inflating the balloon 15. Alternatively, the distal delivery device 7 comprises an elongate flexible member 12 and an inflatable balloon 15 intended to assist the deployment of arms 8. The distal delivery device 7 may comprise a separate inflation line or an integrated inflation line, with an inflation port 16 into the balloon 15. It could also be envisaged that the proximal delivery device 6 comprises the inflation balloon 15 and an inflation line. It is preferred that the balloon 15 is secured to the distal or proximal delivery device 6,7 so that the balloon 15 can be detached from the intracorporeal device 14 and removed from the patient together with the delivery device after successful delivery of the intracorporeal device 4.

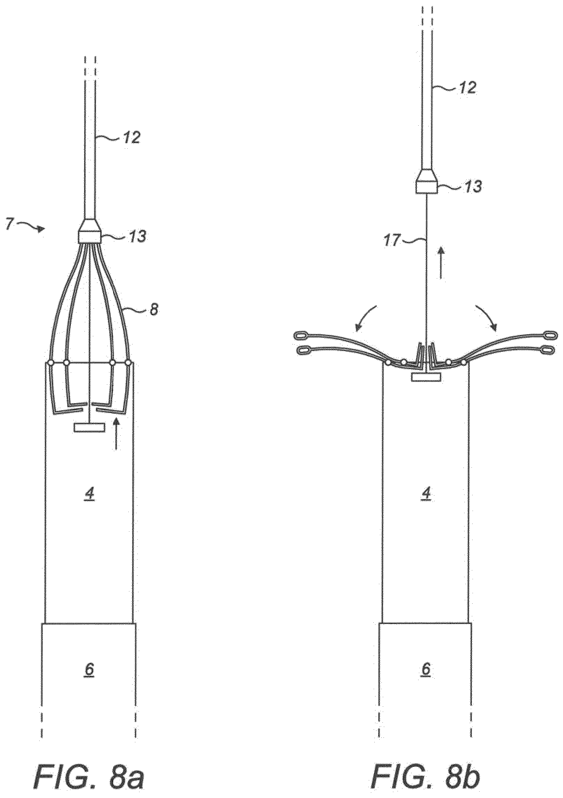

Figures 8a and 8b illustrate a different means for assisting the deployment of the arms 8. The arms 8 are hingedly connected to the intracorporeal device 4. The distal delivery device 7 comprises means 17 for pivoting the arms 8 about the hinges into their working position.

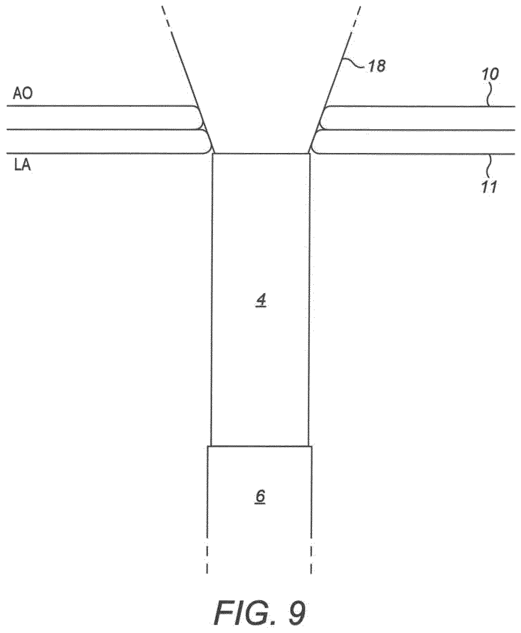

Figure 9 illustrates an intracorporeal device 4 comprising a housing as described above and a distal flow diffusor 18. The diffusor 18 may comprise a plurality of arms or blades 8. In this embodiment, the arms 8 serve the dual purpose of connecting the intracorporeal device 4 to the delivery device and acting as flow diffusor to improve and/or enhance the fluid flow from the pump. Preferably, the diffusor 18 comprises a plurality of blades with an membrane extending between said blades 8. Alternatively, the diffusor 18 may comprise a membrane and be devoid of arms and blades. The diffusor 18 may be connected to the distal delivery device 7 as described above in connection to the connector arms, and be deployed in the same manner.

In the method herein described, the intracorporeal device 4 need not comprise distal expandable arms or blades 8 as described above as long as it comprises means for interconnecting with a delivery device 7 so as to be pulled towards the target site 4 or towards the exit point 3. The interconnection between the intracorporeal device 4 may comprise means such one or more tabs, screwing means, hooks, loops and/or snares and the like.

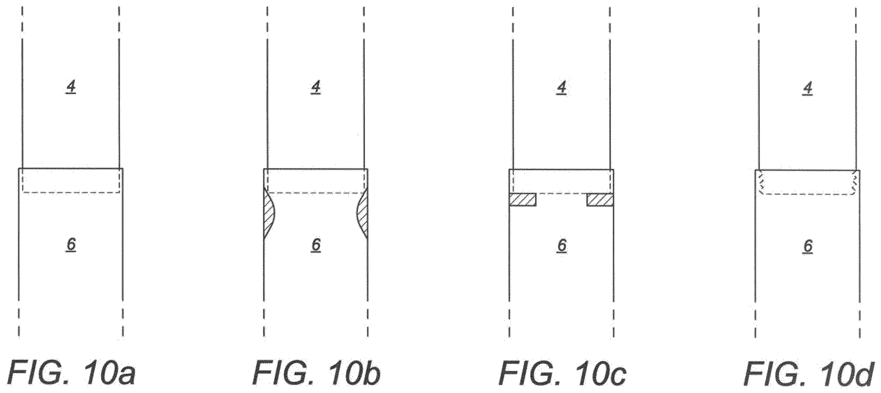

Turning now to the interconnection between the proximal end 4P of the intracorporeal device 4 and the second proximal delivery device 6, figures 10a to 10d show examples of delivery devices 6 comprising a delivery catheter. The delivery catheter 6 may be made of a biocompatible flexible material. The inner diameter of the delivery catheter 6 may be equal or smaller than the outer diameter of the proximal end 4P of the intracorporeal device 4 so as to retain the intracorporeal device 4 therein. This may be achieved by using a resilient material (figure 10a) or by including a portion of narrower diameter (figure 10b). The deliver catheter 6 may comprise one or more ribs or tabs to act as an abutment to assist the pushing of the intracorporeal device 4 (figure 10c). The delivery catheter 6 and the intracorporeal device 4 may comprise complementary connection means, such as screwing means (figure 10d).

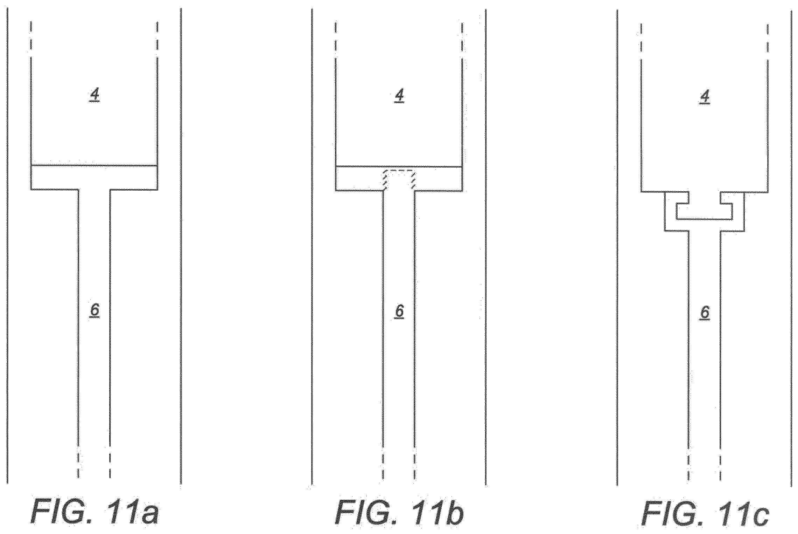

Figures 11a to 11c show examples of delivery devices 6 comprising a wire or cable. When the primary purpose of the delivery device 6 is to push the intracorporeal device 4, then a cable is preferred as it provides suitable rigidity and support, however, it should also have sufficient flexibility to navigate through the patient's anatomy . The cable 6 may be integrally formed or secured to the proximal end of the intracorporeal device 4 (figure 11a), and may for example be a cable for providing the intracorporeal device 4 with electrical energy. It is however preferred that a separate delivery device 6 or cable is provided, because the electrical cable feeding the intracorporeal device needs to be flexible. This is because once implanted in the patient's anatomy, the cable should not mechanically hinder (or be compromised by) the beating of the patient's heart. The cable 6 may be detachably connected to the intracorporeal device 4, for example using screwing means (figure 11b), tabs (figure 11c), hooks and the like. The wire/cable-intracorporeal device combination may be delivered through a delivery catheter or sheath. If other components or intracorporeal devices (such as driveline or battery) are required, then they may be delivered to the target site using the same delivery catheter or sheath.

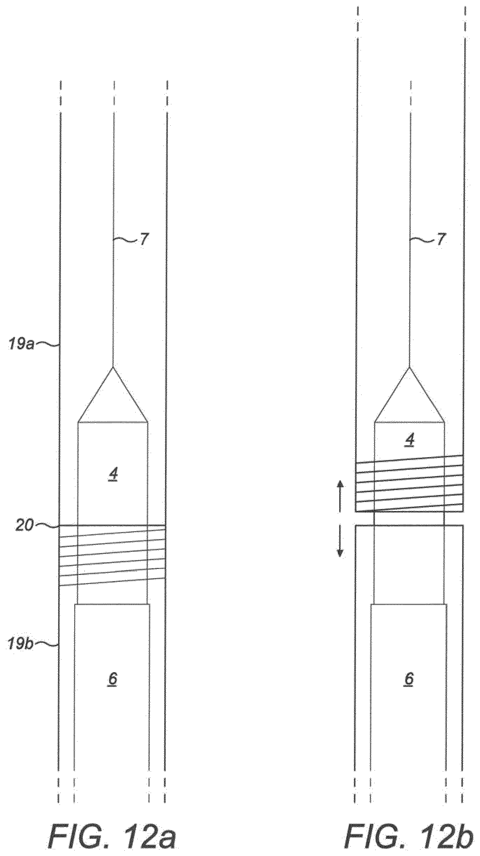

Another device 19 for use in the method herein described is illustrated in figures 12a and 12b. The device 19 comprises a sheath with at least one radial separation line 20 so that the sheath 19 can be separated into two portions 19a and 19b. The radial separation line 19 may comprise a tear line, complementary screwing means, complementary tabs, ribs, hooks and the like. This device 19 is useful as an outer delivery sheath, such as one used to define the device delivery pathway 6. The device 19 may alternatively be used as a device 6,7 for the delivery of intracorporeal device 4. Each portion 19a and 19b may be detachably connected to the intracorporeal device 4 during the delivery process and disconnected after successful delivery.

Within the context of the invention, it is envisaged that either or both delivery devices 6,7 comprise means for remotely controlling the interconnection between the delivery device(s) and the intracorporeal device 4, to remotely guide and manipulate the intracorporeal device and/or to remotely guide and manipulate the end of the delivery device(s) inside the patient.

A method will now be described by way of example with reference to the anterior delivery and implantation of an intracorporeal device 4 for fluid connection between the left atrium LA and the aorta AO, with target implantation site across the roof of the left atrium and the aortic wall.

The first step is the insertion of a guide wire, which can be carried out by means known in the art. An incision or puncture is performed in the groin area of the patient, adjacent the femoral vein or artery. A guide wire is advanced along the femoral vein and up the inferior vena cava and enters the right atrium. A septal puncture between the right and left atrium can also be carried out by means known in the art. A puncture device such as one described in the Applicant's PCT/EP2015/055578 is used to push the left atrium and the aortic wall against each other and puncture both walls.

Known visualisation techniques such as fluoroscopy, and echocardiography d techniques may be used. The inventors have also discovered that techniques for mapping the puncture site can be crucial in hindered and complex target sites such as the heart. This is particularly true when the target site is across two separate anatomical walls, as the puncture wire (or needle and/or stylet) may have a tendency to deflect from the wall surface is not positioned centrally and at a specific angle. The preferred mapping techniques in the context of the present invention include fluoroscopy, Intra-Cardiac Echochardiography techniques (ICE), Trans-Cardiac Echochardiography (TCE), Trans-Thoracic Echochardiography (TTE), Computer Tomography (CT), Magnetic Resonance Imaging (MRI) techniques.

Any of the delivery devices and/or delivery sheath can comprise magnet or magnetic means to accurately position the devices and surround the puncture site. For example, the distal end of the proximal delivery device in the first anatomical compartment and the proximal end of the distal delivery device in the second anatomical compartment may have magnetic coupling or other coupling means so that the two ends meet accurately with each other on either side of the anatomical walls to be punctured.

Once the punctures have been carried out, the guide wire is advanced into the aorta into a subclavian artery where a further puncture is made in the arterial wall. The guide wire exits the patient's body in the patient's neck area. Using methods and devices known in the art, an outer delivery sheath is positioned around the guide wire and along the device delivery pathway 6. In a preferred embodiment, the outer delivery sheath is a device 19 as shown in figure 12 and the radial separation line 20 is positioned adjacent the target site 5, in this case the roof of the left atrium and the aortic wall. The delivery pathway 6 between an entry point 2 and exit point 3 is established.

Outside the patient's body, the intracorporeal device 4 is prepared for insertion. The distal end 4D of the intracorporeal device 4 comprises a plurality of arms 8. The distal delivery device 7 comprises a wire 12 and a tubular receiving portion 13. The distal end of the arms 8 are received in the receiving portion 13 of a distal delivery device 7. In a preferred embodiment, a wire 21 comprising an inflatable balloon 15 is pre-loaded into the distal delivery device 7. The inflatable balloon 15 comprises means for detachably connecting with the distal end of the intracorporeal device 4, in this case screw means 22. In another preferred embodiment, the delivery device 7 does not comprise an inflatable balloon 15 and self-expanding arms 8 are used instead (e.g. shape-memory material, such as nitinol). The proximal delivery device comprises a catheter 6. The distal end of the catheter is connected to the proximal end 4P of the intracorporeal device 4 for example by means of complementary screw means. The intracorporeal device 4 is connected to both delivery devices 6,7 and ready for insertion.

The wire 12 of the distal delivery device 6 is inserted first through the entry point 2 into the outer delivery sheath 19 and pushed forward until it exits the patient at exit point 3. The intracorporeal device 4 is advanced through the outer sheath 19 along the delivery pathway 6 by pushing using the proximal delivery device 6 and/or pulling using the distal delivery device 7, until it reaches the target delivery site 5.

In an alternative embodiment, the intracorporeal device 4 is connected (extracorporeally) to the proximal delivery device 6, inserted through the entry point 2 and advanced along the delivery pathway 6 to the target delivery site 5. The distal delivery device 7 is inserted through the exit point 3 and advanced along the delivery pathway 6 to the target delivery site 5, where it is intracorporeally connected to the intracorporeal device 4. However, the extracorporeal connection of the intracorporeal device 4 to both distal and proximal delivery devices is preferred, as it minimised the number of manipulation within the patient, and therefore the risk of accidents.

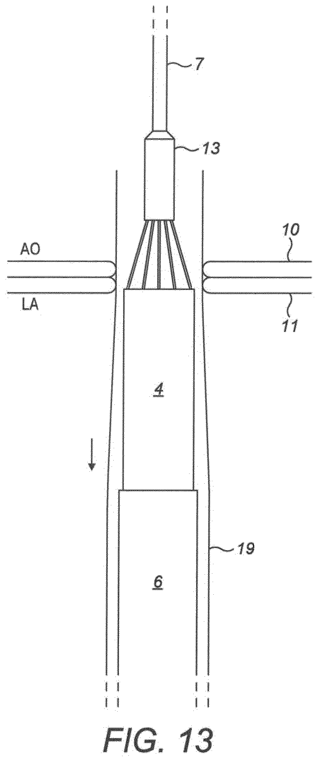

The intracorporeal device 4 is accurately and safely guided and positioned across the anatomical walls (i.e. roof of the left atrium and aortic wall) using the distal delivery device 7 and the proximal delivery device 6. If the outer delivery sheath is a sheath as shown in figure 12, then sheath distal and proximal portions 19a and 19b are separate from each other and the distal portion 19a is pulled out of the patient's body. If the outer delivery sheath is a standard one-piece delivery sheath, then it is pulled back towards the entry point 2, until its distal end is adjacent the target delivery site 5. The intracorporeal device 4 is now ready for implantation (Figure 13).

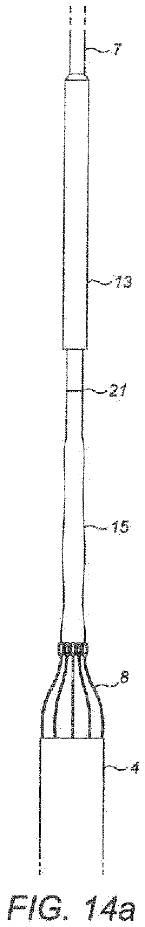

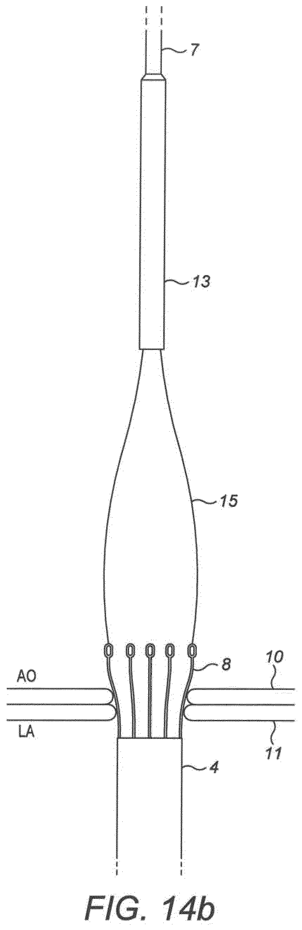

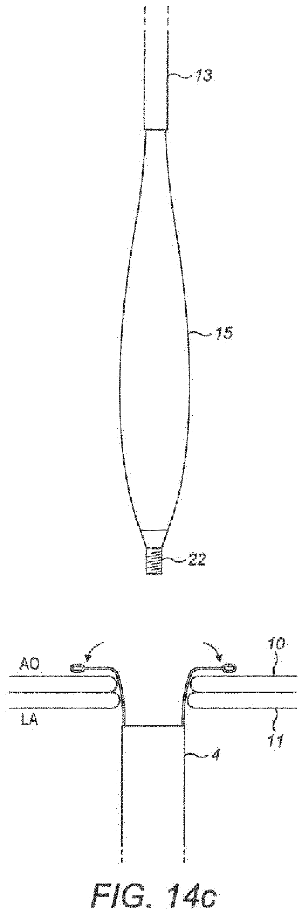

With reference to figures 14a to 14c, the delivery device 7 is pulled back towards the exit point 3 so as to release the ends of the arms 8 of the intracorporeal device 4 (figure 14a). The inflatable balloon 15 is inflated using the inflation line (not shown) so as to assist the deployment of the arms 8 (figure 14b). The arms 8 deploy until they reach their working position, lying against the aortic wall 10. The balloon 15 is deflated and detached from the intracorporeal device 4 by unscrewing screwing means 22. The distal delivery device 7 may now be removed from patient through exit point 3. The proximal delivery device 6 may also be detached from the intracorporeal device 4 and removed from the patient through entry point.

Figures 16a to 16e illustrate a further process for the delivery (or retrieval) of an intracorporeal device 400 according to the present invention. The intracorporeal device 400 comprises at its distal end an expandable distal component 800, shown in a working configuration in figures 16a and 16b and in a delivery/retrieval configuration in figures 16c to 16e. The intracorporeal device 400 is detachably coupled to delivery device 700. In this embodiment, the delivery device 700 comprises a proximal end with a screw means 701 which can be coupled with a complementary screw means (not shown) inside the intracorporeal device 400. Other coupling means may be considered (such as clamping means); however, it has been found that screw means offer the simplest and safest coupling, in that very little lateral movement is involved in the (un-)coupling process. The screw means 701 is located at the distal end, for example of an inner shaft 702, which is slidably coupled to an outer shaft 120. The outer shaft 120 comprises a proximal portion 130 which is arranged and configured to contain, partially or completely, the expandable distal component 800 during the delivery/retrieval process (see figures 16c to 16e in which the distal component 800 is maintained in an unexpanded configuration by proximal portion 130 of the outer shaft 120). The inner shaft 702 may comprises, at its distal end, screwing means (or other coupling means) to detachably couple with an outer shaft retainer 140, for example with a complementary screwing means (not shown) inside the outer shaft retainer 140. In a coupled configuration (see figures 16d and 16e), the outer shaft retainer 140 secures the outer shaft 130 so that the distal component 800 is collapsed in a delivery/retrieval configuration and partially or completely contained within proximal component 130 of outer shaft 120. At its distal end, the outer shaft retainer 140 may be detachably coupled to an elongated guiding means 150, such as a thread, cable, wire, guide wire and the like. The outer shaft retainer may comprise a swivel 160 for coupling with the guiding means 150, whilst enabling movement and control of the outer shaft retainer 140 relative to the guiding means 150. Thus, a delivery device 7, 700 according to the present invention may comprise any one or more of the features described in this paragraph.

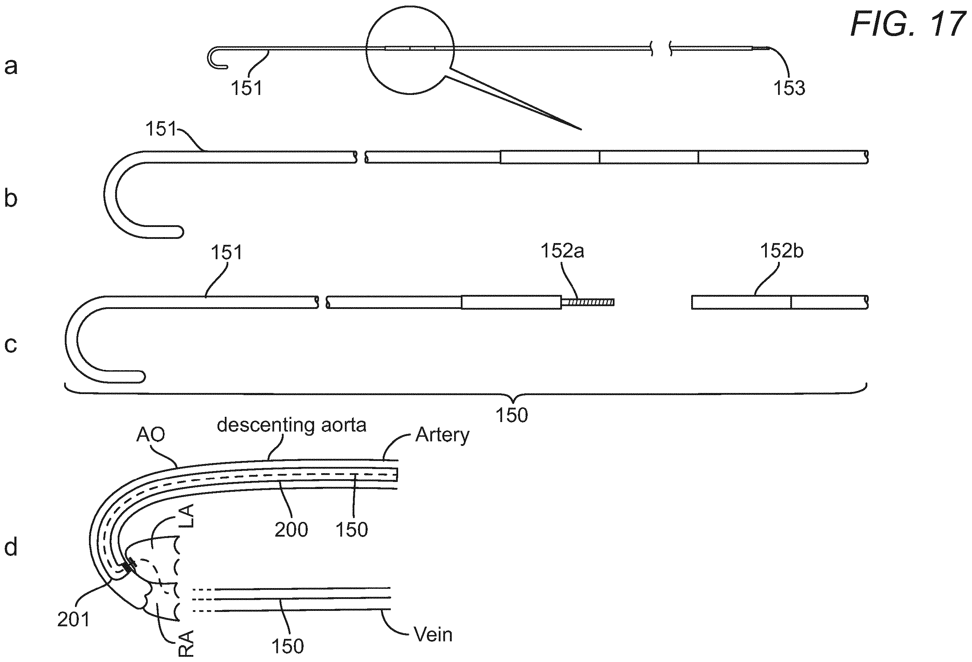

Figures 17a to 17c illustrate a guide wire 150 for use in the processes described herein. In a process as herein described, a guide wire is inserted in the femoral vein and move along the inferior vena cava, up to the right atrium. The guide wire crosses the atrial septum into the left atrium and continues into the aorta. A snare is advanced from the arterial system to capture the distal tip of the guide wire and pull it out of the artery at a desired location. The snare used in this process can be a tri-lobe loop snare or other gooseneck type snare or and like. During the snaring step, the snare grasps the soft and flexible distal end of the guide wire, and the guide wire can become damaged or kinked at the grasp location. This damaged or kinked section of the guide wire can make it difficult to use or unusable. For example, it becomes difficult to pass other components such as catheters and sheath over the damaged section. Once damaged, a standard guide wire would need to be withdrawn from the patient's body, and replaced by a new guide wire. The inventors propose a guide wire 150 comprising a replaceable distal end 151. The distal end is detachably coupled to the guide wire 150, for example using complementary screwing means 152a, 152b. The guide wire 150 may comprise a proximal means 153 for coupling with a catheter or sheath, for example a screwing means. This guide wire 150 is particularly advantageous in that it enter the patient from one entry point 2 and exits through the exit point 3. Once the end of the guide wire is externalised from the patient, the distal end 151 of the guide wire 150 can be detached (or unscrewed) and replaced with a new end 151, without the need for withdrawing, replacing and refitting the whole guide wire through the patient's body.

The inventors propose a further new device 200 (as shown in figure 17d) for use in the context of the present invention. Figure 17d shows a catheter 200 comprising a curved distal tip 210 (i.e. distal relative to the direction of insertion). The curvature of the distal tip 210 enables the positioning of the catheter 200 at a suitable angle for the manipulation of the intracorporeal device(s), delivery or retrieval devices. The curvature of the distal tip 210 may be adjusted in order to achieve the optimum angle for manipulation. This modified catheter 200 is advantageous in the context of a left atrium - aorta procedure, where access and therefore manipulation is difficult over the minor and major curves of the aorta. This modified catheter 200 is also particularly advantageous in the context of a groin-to-groin procedure (or other procedure where the entry point is adjacent to the exit point) in that the manipulation of the various devices and components of the present system can add strain onto the patient's anatomy, for example onto the curves of the aorta. The use of this modified catheter 200 allows a relief of said strain and lowers the risk of injury.

Once the guide wire 150 is implanted along the device delivery pathway within the patient, the modified catheter 200 may be advanced along said guide wire 150 for example from the exit point 3 in the groin area through the femoral artery, up the descending aorta, then over the aortic arch to be positioned at a suitable angle at the target implantation site. In a preferred embodiment, a delivery device 700 such as that shown in figures 16a to 16e is advanced through and exits the modified catheter 200, guided and supported by guide wire 200. The delivery device 700 is positioned at the correct location and positioned at the correct angle for interaction between the delivery device 700, the guide wire 150, the primary and secondary connector means 40,80 at the target implantation site.

In the above-described embodiments, the distal component 8,800 of the intracorporeal device 4,400 may act as a connector or anchor to secure the intracorporeal device 4,400 to anatomical walls, such as the roof of the left atrium 11 and the aortic wall 10.

Thus, the inventors propose an intracorporeal system comprising a primary connector means 50 for contacting two or more anatomical walls together; and an intracorporeal device 40 comprising a secondary connector means 80 for securing the intracorporeal device 40 to the primary connector means 50. The present invention also relates to said intracorporeal device 40 comprising a secondary connector means 80 for securing the intracorporeal device 40 to a primary connector means 50 for contacting two or more anatomical walls together. The primary connecting means 50 may comprise a neck 51 which in a working configuration sits across the anatomical walls, a first set of arms 52 which in a working configuration lay substantially along the wall of the distal anatomical compartment (e.g. the aorta), and a second set of arms 53 which in a working configuration lay substantially along the wall of the proximal anatomical compartment (e.g. the left atrium). Figure 18a illustrates a primary connector means positioned in a working configuration across two anatomical walls; figure 18b illustrates the secondary connector means coupled in a working configuration with the primary connector means; figure 18c is a schematic view of the primary and secondary connector means from the distal anatomical compartment (e.g. the aorta), figure 18d is a schematic view of the primary connector means and the intracorporeal device from the proximal anatomical compartment (e.g. the left atrium).

In a delivery/retrieval configuration, the arms 52, 53 extend substantially longitudinally from the proximal and distal end of the neck 51. Examples of primary connector means 50 may be found in the Applicant's other patent applications, PCT/EP2015/055578, PCT/EP2016/082889 and PCT/EP2017/050275. In a preferred embodiment, the arms 51 and/or 52 are curved away from the anatomical wall(s) in order to adequately support the anatomical walls whilst preventing tissue growth onto the arms 51,52.

The present invention has been described in connection to a device delivery process. It is however envisaged that the present invention may be used in connection to a device retrieval process. In the retrieval process, the guide wire and outer sheath are positioned as described above in connection to the delivery process. The distal delivery retrieval device 7 (now distal retrieval device 7) is inserted through the exit point 3 and connected to the distal end 4D of the intracorporeal device 4. The proximal delivery device 7 (now proximal retrieval device 6) is inserted through the entry point 2 and connected to the proximal end 4D of the intracorporeal device 4. The intracorporeal device 4 can be accurately and safely dislodged from the implantation position, and removed from the patient's body.

In another embodiment, the intracorporeal device 4 may be a connector 40 as described in the Applicant's PCT/EP2015/055578, comprising a neck 41 for fluid passage between two compartments LA, AO, a first set of arms 8a destined to lie against the aortic wall 10 in a working configuration, and a second set of arms 8b destined to lie against the roof of the left atrium 11 in a working configuration. This connector 40 is particularly useful when used to anchor a second intracorporeal device such as the pump described above, where said pump does not comprise an integral set of arms (connector).

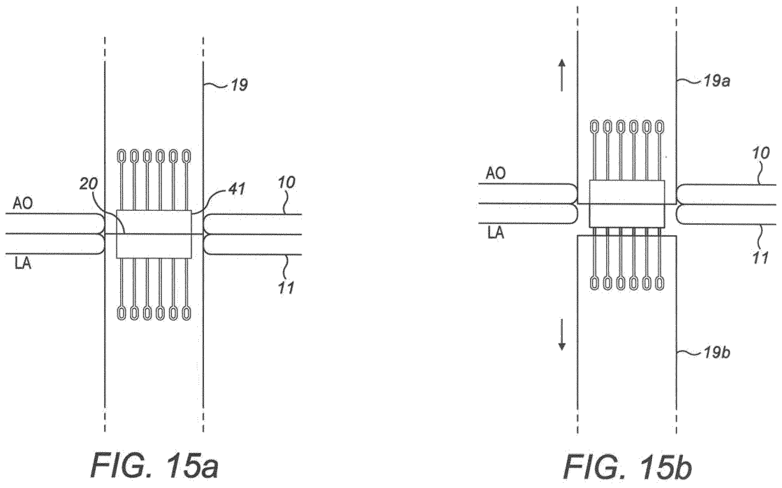

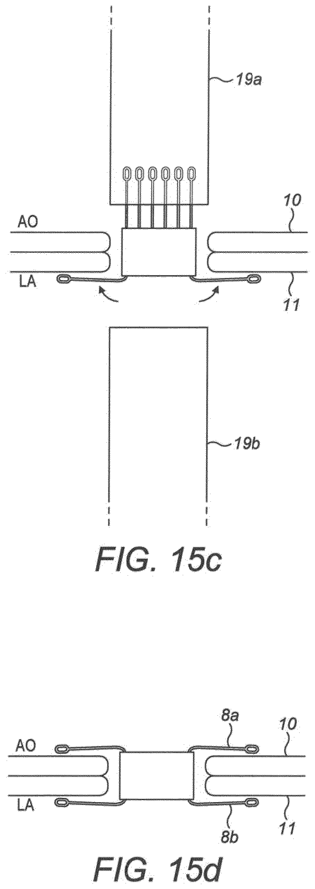

With reference to figure 15a to 15d, the anchor 40 is delivered to the target site 5 through a delivery sheath 19 with a radial separation mechanism 20. The neck 41 and the separation line 20 are positioned across the anatomical walls 10, 11 (figure 15a). The catheter portions 19a and 19b are separated from each other (figure 15b). Proximal catheter portion 19b is pulled back so as to release the atrial arms 8b. The atrial arms 8b expand to lie into their working configuration against the roof of the left atrium 11. Then distal catheter portion 19a is pulled back so as to release the aortic arms 8a. The aortic arms 8a expand to lie into their working configuration against the aortic wall 10. The connector 40 is now implanted into its working configuration and the intracorporeal device 4 (e.g. pump) can now be delivered using the method and devices according to the present invention.

In the method herein described, the anatomical walls are preferably held together by a connector (integral or separate connector) so as to minimise the risk of blood leakage into the inter-compartmental space between the left atrium and the aorta. In addition, the use of the connector is also advantageous in that the walls are less likely to slide or move during the process of implanting an intracorporeal device 4 across the two anatomical walls. Where the intracorporeal device 4 is to be implanted without a connector 40 (integral or separate connector), preferably the method comprises the additional step of suturing and/or stapling the two anatomical walls to each other by means known in the art. Thus, no additional connector device (integral or separate connector) is required.

Thus, from the above description, it can be seen that a versatile and accurate method for the delivery or retrieval of an intracorporeal device is provided. The present invention also provides devices for use in said method.

## Claims

1. An intracorporeal device for delivery into a patient, **characterised by** said intracorporeal device having a proximal end (4P) adapted to be detachably coupled with a proximal transcatheter delivery device and a distal end (4D) adapted to be detachably coupled with a distal transcatheter delivery device, and further comprising an integrated expandable distal component, said distal component being able to be expanded from a delivery configuration to a working configuration, the delivery configuration being the configuration in which the intracorporeal device can be coupled to the delivery device, whereas in the working configuration, the intracorporeal device is detached from the delivery device.

2. The intracorporeal device according to claim 1, wherein, in the working configuration, the integrated expanded distal component is arranged and configured to secure the intracorporeal device to one or more anatomical walls.

3. The intracorporeal device according to claim 1, wherein, in the working configuration, the integrated expanded distal component is a flow diffusor.

4. The intracorporeal device according to claim 1, wherein the integrated expandable distal component comprises a plurality of expandable arms and/or blades (8).

5. The intracorporeal device according to claim 4, wherein the integrated expandable distal component comprises a membrane extending between the arms and/or blades.

6. The intracorporeal device according to claim 1, further comprising means for assisting the expansion of the integrated expandable distal component into its working configuration.

7. The intracorporeal device according to claim 6, wherein the assisting means comprises an inflatable balloon (15).

8. The intracorporeal device according to claim 1, comprising a detachable coupling component, said distal component being able to be expand from a delivery configuration to a working configuration.

9. The intracorporeal device according to claim 8, wherein the detachable component comprises a cap and/or a sheath.

10. The intracorporeal device according to claim 8, wherein the detachable component is coupled to the delivery device.

11. The intracorporeal device according to claim 1, comprising an integrated expandable distal component, said distal component being able to be retracted from a working configuration to a retrieval configuration.

12. The intracorporeal device according to claim 11, wherein, in the retrieval configuration, the integrated expanded distal component is arranged and configured to release the intracorporeal device from said one or more anatomical walls.

13. The intracorporeal device according to claim 1, comprising a detachable coupling component, said distal component being able to be retracted from a working configuration to a retrieval configuration.

## Patentansprüche

1. Intrakorporale Vorrichtung zur Abgabe in einen Patienten, **dadurch gekennzeichnet, dass** die intrakorporale Vorrichtung Folgendes aufweist: ein proximales Ende (4P), das zur trennbaren Verbindung mit einer proximalen Transkatheter-Abgabevorrichtung geeignet ist, und ein distales Ende (4D), das zur trennbaren Verbindung mit einer distalen Transkatheter-Abgabevorrichtung geeignet ist, und die weiter eine integrierte expandierbare distale Komponente umfasst, wobei die distale Komponente von einer Abgabekonfiguration zu einer funktionierenden Konfiguration expandiert werden kann, wobei die Abgabekonfiguration die Konfiguration ist, in der die intrakorporale Vorrichtung mit der Abgabevorrichtung verbunden sein kann, wohingegen in der funktionierenden Konfiguration die intrakorporale Vorrichtung von der Abgabevorrichtung getrennt ist.

2. Intrakorporale Vorrichtung nach Anspruch 1, wobei in der funktionierenden Konfiguration die integrierte expandierte distale Komponente zur Befestigung der intrakorporalen Vorrichtung an einer oder mehreren anatomischen Wänden angeordnet und konfiguriert ist.

3. Intrakorporale Vorrichtung nach Anspruch 1, wobei in der funktionierenden Konfiguration die integrierte expandierte distale Komponente ein Flussverteiler ist.

4. Intrakorporale Vorrichtung nach Anspruch 1, wobei die integrierte expandierbare distale Komponente eine Vielzahl von expandierbaren Armen und/oder Flügeln (8) umfasst.

5. Intrakorporale Vorrichtung nach Anspruch 4, wobei die integrierte expandierbare distale Komponente eine Membran umfasst, die sich zwischen den Armen und/oder Flügeln erstreckt.

6. Intrakorporale Vorrichtung nach Anspruch 1, die weiter Mittel zur Unterstützung der Expansion der integrierten expandierbaren distalen Komponente zu ihrer funktionierenden Konfiguration umfasst.

7. Intrakorporale Vorrichtung nach Anspruch 6, wobei das Unterstützungsmittel einen aufblasbaren Ballon (15) umfasst.

8. Intrakorporale Vorrichtung nach Anspruch 1, die eine trennbare Verbindungskomponente umfasst, wobei die distale Komponente von einer Abgabekonfiguration zu einer funktionierenden Konfiguration expandiert werden kann.

9. Intrakorporale Vorrichtung nach Anspruch 8, wobei die trennbare Komponente eine Kappe und/oder eine Hülse umfasst.

10. Intrakorporale Vorrichtung nach Anspruch 8, wobei die trennbare Komponente mit der Abgabevorrichtung verbunden ist.

11. Intrakorporale Vorrichtung nach Anspruch 1, die eine integrierte expandierbare distale Komponente umfasst, wobei die distale Komponente von einer funktionierenden Konfiguration zu einer Rückholkonfiguration zurückgezogen werden kann.

12. Intrakorporale Vorrichtung nach Anspruch 11, wobei in der Rückholkonfiguration die integrierte expandierte distale Komponente zur Freigabe der intrakorporalen Vorrichtung von der einen oder den mehreren anatomischen Wänden angeordnet und konfiguriert ist.

13. Intrakorporale Vorrichtung nach Anspruch 1, die eine trennbare Verbindungkomponente umfasst, wobei die distale Komponente von einer funktionierenden Konfiguration zu einer Rückholkonfiguration zurückgezogen werden kann.

## Revendications

1. Dispositif intracorporel destiné à être délivré dans un patient, **caractérisé en ce que** ledit dispositif intracorporel a une extrémité proximale (4P) conçue pour être couplée de manière détachable avec un dispositif de délivrance de transcathéter proximal et une extrémité distale (4D) conçue pour être couplée de manière détachable avec un dispositif de délivrance de transcathéter distal, et comprenant en outre un composant distal déployable intégré, ledit composant distal étant capable de se déployer d'une configuration de délivrance à une configuration de travail, la configuration de délivrance étant la configuration dans laquelle le dispositif intracorporel peut être couplé au dispositif de délivrance, alors que dans la configuration de travail, le dispositif intracorporel est détaché du dispositif de délivrance.

2. Dispositif intracorporel selon la revendication 1, dans lequel, dans la configuration de travail, le composant distal déployé intégré est agencé et configuré pour fixer le dispositif intracorporel à une ou plusieurs parois anatomiques.

3. Dispositif intracorporel selon la revendication 1, dans lequel, dans la configuration de travail, le composant distal déployé intégré est un diffuseur d'écoulement.

4. Dispositif intracorporel selon la revendication 1, dans lequel le composant distal déployable intégré comprend une pluralité de bras et/ou de lames déployables (8).

5. Dispositif intracorporel selon la revendication 4, dans lequel le composant distal déployable intégré comprend une membrane s'étendant entre les bras et/ou les lames.

6. Dispositif intracorporel selon la revendication 1, comprenant en outre un moyen destiné à aider le déploiement du composant distal déployable intégré en sa configuration de travail.

7. Dispositif intracorporel selon la revendication 6, dans lequel le moyen d'aide comprend un ballonnet gonflable (15).

8. Dispositif intracorporel selon la revendication 1, comprenant un composant de couplage détachable, ledit composant distal étant capable de se déployer d'une configuration de délivrance à une configuration de travail.

9. Dispositif intracorporel selon la revendication 8, dans lequel le composant détachable comprend un capuchon et/ou une gaine.

10. Dispositif intracorporel selon la revendication 8, dans lequel le composant détachable est couplé au dispositif de délivrance.

11. Dispositif intracorporel selon la revendication 1, comprenant un composant distal déployable intégré, ledit composant distal étant capable de se rétracter d'une configuration de travail à une configuration de récupération.

12. Dispositif intracorporel selon la revendication 11, dans lequel, dans la configuration de récupération, le composant distal déployé intégré est agencé et configuré pour libérer le dispositif intracorporel desdites une ou plusieurs parois anatomiques.

13. Dispositif intracorporel selon la revendication 1, comprenant un composant de couplage détachable, ledit composant distal étant capable de se rétracter d'une configuration de travail à une configuration de récupération.
